# EUROPÄISCHE PATENTSCHRIFT

(11) **EP 1 952 799 B1**
(45) Veröffentlichungstag und Bekanntmachung des Hinweises auf die Patenterteilung: **11.01.2017**
(21) Anmeldenummer: 08103572.7
(22) Anmeldetag: 01.11.2005
(51) Int. Cl.: A61K 8/97, A61Q 19/08, A61K 8/73

(54) **KOSMETISCHE ZUBEREITUNG MIT HYALURONSÄURE UND SAPONINEN ZUR BEHANDLUNG VON HAUTALTERUNGSERSCHEINUNGEN**
COSMETIC PREPARATION WITH HYALURONIC ACID AND SAPONINS TO TREAT SIGNS OF SKIN AGEING
PREPARATION COSMETIQUE COMPRENANT L'ACIDE HYALUONIQUE POUR TRAITER LES SIGNES DE VIEILLISSEMENT

(30) Priorität: 16.03.2005 DE 102005012554
(43) Veröffentlichungstag der Anmeldung: 06.08.2008
(62) Teilanmeldung aus: 05801470.5
(73) Patentinhaber: Beiersdorf AG, 20245 Hamburg (DE)
(72) Erfinder: Gallinat, Stefan, D-22880 Wedel (DE); Mummert, Christopher, 29553, Bienenbüttel (DE); Bürger, Anette, 22301, Hamburg (DE); Filbry, Alexander, 22459, Hamburg (DE); Fänger, Sabine, 22763, Hamburg (DE)

(56) Entgegenhaltungen:
- EP-A- 0 852 946
- FR-A- 2 746 316
- FR-A- 2 767 059
- FR-A- 2 791 260
- FR-A- 2 848 116
- GB-A- 2 259 015
- JP-A- 8 217 660
- US-A- 5 663 160
- US-A1- 2003 165 456
- PATENT ABSTRACTS OF JAPAN Bd. 1999, Nr. 05, 31. Mai 1999 (1999-05-31) & JP 11 035445 A (OSAKA YAKUHIN KENKYUSHO:KK), 9. Februar 1999 (1999-02-09)
- "Horsetail", 1 June 2007 (2007-06-01), 20070601, PAGE(S) 1 - 4, XP007922968,
- SCHANEBERG B T ET AL: "An improved HPLC method for quantitative determination of six triterpenes in Centella asiatica extracts and commercial products B. T", DIE PHARMAZIE, GOVI VERLAG PHARMAZEUTISCHER VERLAG GMBH, ESCHBORN, DE, vol. 58, 1 January 2003 (2003-01-01), pages 381-384, XP007922969, ISSN: 0031-7144
- "Equisetum arvense" In: Karl Hiller/Matthias F. Melzig: "Lexikon der Arzneipflanzen und Drogen", 31 December 2010 (2010-12-31), Spektrum Akademischer Verlag, Heidelberg, Deutschland ISBN: 078-3-8274-2053-4 pages 214-214,
- 'Teedrogen. Ein Handbuch für die Praxis auf wissenschaftlicher Grundlagen', WISSENSCHAFTLICHE VERLAGSGESELLSCHAFT MNH, STUTTGART, DEUTSCHAND vol. 'Equiseti herba', Seiten 204 - 204

## Beschreibung

Die vorliegende Erfindung betrifft kosmetische Zubereitungen mit einer Wirkstoffkombination aus Hyaluronsäure und Saponinen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut. Um der Haut ein makelloses Aussehen zu verleihen, bedarf es der regelmäßigen Reinigung und Pflege.

Zur Pflege der Haut werden den Verbrauchern heutzutage eine Vielzahl von kosmetischen Zubereitungen, meist in Form von Cremes und Lotionen d.h. als Emulsion angeboten. Produkte, welche die Alterungserscheinungen der Haut (insbesondere das Auftreten von Fältchen und Falten) temporär oder dauerhaft verzögern oder beseitigen, haben dabei eine stetig wachsende Bedeutung. Neben Wasser zur Hautbefeuchtung sowie Ölen und Lipiden zur Rückfettung der Haut enthalten derartige Hautpflegeprodukte eine Vielzahl an Wirk- Hilfs- und Zusatzstoffen.

Die "reife Haut" älterer Menschen unterscheidet sich von "normaler Haut" jüngerer in einer Vielzahl von Symptomen. Sie ist in der Regel trockener und zeigt eine ungleichmäßige Verhornung. Durch ihr fehlendes Wasserbindevermögen in der Lederhaut entstehen tiefe Falten. Die Neigung der Oberhaut zu blasigen Abhebungen ist erhöht. Das Bild der Altershaut entwickelt sich beim genetischen Altern ähnlich wie bei chronischer Umweltschädigung wie sie zum Beispiel durch übermäßige UV-Exposition hervorgerufen wird.

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
a) Sichtbare Gefäßerweiterungen (Teleangiektasien, Cuperosis);
b) Schlaffheit und Ausbildung von Falten;
c) lokale Hyper-, Hypo- und Fehlpigmentierungen (z.B. Altersflecken);
d) vergrößerte Anfälligkeit gegenüber mechanischem Stress (z.B. Rissigkeit)
e) Abnahme des Kollagengehaltes der Haut (z.B. durch verminderte Neusynthese und/oder durch verstärkten Abbau)
f) Störungen des Glykosaminoglykan und Elastinstoffwechsels.

Die Hautforschung hat in den vergangenen Jahren eine Vielzahl an Wirkstoffen entwickelt bzw. entdeckt, mit denen sich Hautalterungserscheinungen kosmetisch behandeln lassen und der optisch wahrnehmbare Hautalterungsprozess verlangsamen läßt.

Herkömmliche Hautpflegeprodukte zur Prophylaxe und Behandlung von Hautalterungssymptomen weisen jedoch den Nachteil auf, dass sich diese Wirkstoffe in der Regel nur schwer und in ungenügenden Mengen in kosmetische Formulierungen einarbeiten lassen. Ferner tritt nach dem Stande der Technik regelmäßig der Nachteil auf, dass sich Kombinationen von Wirkstoffen nur schwer in die Zubereitungen einarbeiten lassen, da die Wirkstoffe nicht nur Unverträglichkeiten gegenüber der "Trägerzubereitung" sondern auch untereinander aufweisen können.

Es war die Aufgabe der vorliegenden Erfindung, eine neue, stabile und kosmetisch wirksame Hautpflegezubereitung zur Prophylaxe und Behandlung von Hautalterungserscheinungen, insbesondere Fältchen und Falten zu entwickeln. Insbesondere sollte durch die Hautpflegezubereitung die Hautfeuchtigkeit erhöht werden.

Überraschend gelöst werden die Aufgaben durch eine kosmetische Zubereitung in Form einer O/W-Emulsion enthaltend eine Wirkstoffkombination aus 0,001 bis 3 Gewichts-% Hyaluronsäure und 0,01 bis 4 Gewichts-% Saponinen, wobei sich die Gewichtsangaben auf das Gesamtgewicht der Zubereitung beziehen, sowie enthaltend Wasser und ein Glycol mit einem log P Wert zwischen -3,6 und 1 dadurch gekennzeichnet, das die Zubereitung eine Lipidphase mit einer Gesamtpolarität von weniger als 3C mN/m enthält.

Zwar kennt der Stand der Technik die US 2003/165456, FR 2848116, FR 2791260, FR 2746316, FR 2767059, EP 0852946, GB 2259015, JP11 035445 und JP 08 217660, doch konnten diese Schriften nicht den Weg zur vorliegenden Erfindung weisen.

Ferner werden die Aufgaben überraschend gelöst durch die Verwendung einer solchen kosmetischen Zubereitung zur nicht therapeutischen Prophylaxe und/oder Behandlung von Hautalterungserscheinungen, insbesondere von Fältchen und Falten.

Es hat sich dabei als erfindungsgemäß besonders vorteilhaft herausgestellt, wenn das Gewichtsverhältnis von Hyaluronsäure zu Saponinen von 1:1 bis 1:10 beträgt.

Vorteilhafte Ausführungsformen der Erfindung sind dadurch gekennzeichnet, dass die Zubereitung Saponine aus Glycosiden von Triterpensapogeninen neben gegebenenfalls weiteren Saponinen aus Glycosiden von Steroidsapogeninen enthält

Hyaluronsäure ist ein im Glaskörper von Augen, der Synovialflüssigkeit der Gelenke und in der Haut vorkommendes Glykosaminoglykan, das zusammen mit den Chondroitinsulfaten und Dermatansulfat ein Bestandteil aller Bindegewebe (außer Augenhornhaut) ist.

Hyaluronsäure ist eine hochmolekulare Verbindung mit M_{R} zwischen 50000 und mehreren Millionen. Grundbaustein der Hyaluronsäure ist ein aus D-Glucuronsäure und *N*-Acetyl-D-glucosamin in β-(1 →3)-glykosidischer Bindung aufgebautes Aminodisaccharid, das mit der nächsten Einheit β-(1 →4)-glykosidisch verbunden ist:

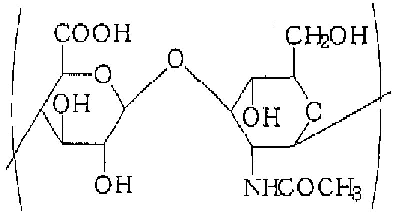

Das Natriumsalz der Hyaluronsäure wird als Moisturizer (Feuchthaltemittel) für die Herstellung kosmetischer Mittel verwendet (Römpp online Lexikon Version 2.5, 2004).

Als Saponine (von "Sapo" latein. Seife) bezeichnet man eine Gruppe von im allgemeinen pflanzlichen Glykosiden, die als oberflächenaktive Verbindungen in Wasser kolloidale, seifenartige Lösungen bilden.

Die Saponine werden nach der Art ihrer Aglykone, der Sapogenine, in Triterpen- Saponine und Steroid- Saponine unterteilt. Der Kohlenhydrat-Anteil kann aus bis zu 11 Monosaccharid-Resten (meistens D-Glucose, D-Galactose, L-Rhamnose, L-Arabinose, D-Xylose, D-Fucose, D-Glucuronsäure) bestehen.

Die wichtigsten in Lebensmitteln vorkommenden Saponine, die Oleanolsäure- Saponine (Zuckerrübe), Glycyrrhizin (Süßholzwurzel) und die Sojabohnen- Saponine, gehören der Reihe der Triterpen- Saponine an.

In der Kosmetik werden Saponine als Netz- und Dispergiermittel bzw. Schaumbildner bei Zahnpulvern, Mundwässern u. Shampoos eingesetzt (Römpp online Lexikon Version 2.5, 2004).

Die erfindungsgemäßen Wirkstoffe sind dem Kosmetik- Fachmann zwar an sich bekannt, doch ist es bisher nicht gelungen, Saponine und insbesondere die Kombination aus Hyaluronsäure und Saponinen stabil und in einer wirksamen Form in kosmetische Zubereitungen, insbesondere Emulsionen, einzuarbeiten. So trat bei der Kombination aus den beiden Wirkstoffen nach dem Stand der Technik immer das Problem auf, dass die homogene Einarbeitung der beiden Rohstoffe nicht nach einem Standardverfahren möglich war. Somit war es auch nicht möglich, eine konstante Wirkstoffkonzentration zu gewährleisten, besonders über einen längeren Lagerzeitraum hinweg. Beide Rohstoffe können nicht über die Lipidphase eingearbeitet werde, da Sie hydrophile Molekülbestandteile enthalten. Aber eine Einararbeitung der beiden Rohstoffe über die Wasserphase ist ebenfalls nicht möglich, da sich die Saponine und Hyaluronsäure nicht gleichzeitig homogen in der Wasserphase lösen/dispergieren lassen, ohne Trübungen/ Kristalle zu bilden.

Das Problem konnte nun erfindungsgemäß gelöst werden, indem ein besonderes Verfahren zur Herstellung einer kosmetischen Zubereitung, die sowohl Saponine als auch Hyaluronsäure enthält, erarbeitet wurde: die Saponine bzw. der die Saponine enthaltende Pflanzenextrakt wird hierbei zunächst mit mindestens der vierfachen Menge an einem Glykol mit einem log P Wert zwischen -3,6 und 1 gemischt und unter ständigem Rühren sowie Erwärmung gelöst. Anschließend wird die Lösung der möglichst noch heißen Präemulsion zugegeben. Alternativ kann das Saponin auch in die Wasserphase der Zubereitung, welche mindestens das Vierfache der Saponinmenge an entsprechendem Glykol enthält, eingearbeitet werden: unter Erwärmung und ständigem Rühren wird die schlecht lösliche Substanz eingerührt und dispergiert und anschließend mit der Fettphase, welche die Emulgatoren enthält, vereinigt. In beiden Fällen erwies es sich als vorteilhaft, die Hyaluronsäure separat in einem Vielfachen an Wasser anzurühren und vorzuquellen, so daß ein Gel entsteht. Dieses wird der nicht mehr ganz heißen Emulsion unter Rühren zugegeben.

Als erfindungsgemäß bzw. erfindungsgemäße Zubereitungen werden im Rahmen der vorliegenden Erfindung sowohl die erfindungsgemäßen Zubereitungen an sich als auch die nach dem erfindungsgemäßen Verfahren hergestellten Zubereitungen als auch die erfindungsgemäß verwendeten Zubereitungen verstanden.

Es ist erfindungsgemäß von Vorteil, wenn die erfindungsgemäße Zubereitung Saponine auf Basis von Triterpensapogeninen enthalten. Bei der Verwendung von Sojasaponin sind das besonders Saponine aus Sojasapogenol A oder Sojasagpogenol B. Aber es können gegebenenfalls auch Saponine auf Basis von Steroidsapogeninen enthalten sein.

Die erfindungsgemäßen Leguminosenextrakte (Fam. Fabaceae) sind vorzugsweise aus Vertretern der Gattungen Abrus, Anagyris, Andira, Anthyllis, Arachis, Aspalathus, Astragalus, Baptistia, Canavalia, Castanospermum, Cicer, Crotalaria, Cyamopsis, Cytisus, Derris, Dipteryx, Galega, Genista, Glycine, Glycyrrhiza, Gymnoclamdus, Indigofera, Laburnum, Lathyrus, Lens, Lespedeza, Medicago, Melilotus, Mucuna, Myroxylon, Ononis, Oxytropis, Phaseolus, Physostigma, Piptadenia, Psicidia, Pisum, Pterocarpus, Robinia, Sophora, Trifolium, Trigonella, Ulex, Vigna, Vicia und Wisteria gewählt.

Besonders geeignet sind hierbei Extrakte aus Soyabohne (Glycine Soja), Saubohne (Vicia faba), Gartenbohne (Phaseolus vulgaris), Linamarin (Phaseolus lunatus), Mungobohne (Phaseolus aureus und Vigna radiata ), Linse (Lens culinaris), Luzerne (Medicago sativa), Chinesischer Traganth (Astragalus membranaceus), Erbse (Pisum sativum), Urdbohne (Vigna mungo), Mattenbohne (Vigna angularis), Süßholz (Glycyrrhiza glabra ), Erdnuß (Arachis hypogaea), Schnurbaumwurzel (Sophora favescens), Geißraute (Galega officinalis), Kichererbse (Cicer arietinum), Fahnenwicke (Oxytropis), Bengalischer Hanf (Crotalaria juncea), Japanischer Blauregen (Wisteria floribunda) und Weißklee (Trifolium repens).

Werden die erfindungsgemäßen Saponine in Form eines Sojaextraktes eingesetzt, so ist es erfindungsgemäß von Vorteil, wenn der Soyaextrakt auf den Sapogeningehalt standardisiert ist. Soyaextrakte enthalten als Naturprodukte eine Vielzahl an Verbindungen, deren wichtigste Vertreter die Fette, Kohlenhydrate, Proteine, Isoflavone, Lecithine und die Saponine sind. Je nach Extraktionsverfahren können die Extrakte unterschiedliche Inhaltsstoffe enthalten. So gibt es Sojaöle oder solche Sojaextrakte, die mind. 90% Isoflavonoide enthalten. Erfindungsgemäß von Vorteil sind diejenigen Soyaextrakte, die einen hohen Gehalt an Saponinen aufweisen. Diese sollten eine Anreicherung an Saponin im Vergleich zum Gehalt in der Soyabohne darstellen. Die Soyabohne enthält ca. 6,5 mg/g Saponine. Der Extrakt sollte mindestens 10mg/g, besonders vorteilhaft aber sogar über 100mg/g Saponin enthalten. Ein Beispiel für einen erfindungsgemäß geeigneten Extrakt ist der Soybean Germ Extract von Lucas Meyer Beauty Essentials mit einem ungefähren Saponingehalt von 140mg/g. Es handelt sich hierbei um ein gelbliches Pulver mit einem leichten Eigengeruch nach gerösteten Nüssen.

Es ist erfindungsgemäß bevorzugt, wenn die erfindungsgemäße Zubereitung in Form einer Emulsion vorliegt und erfindungsgemäß besonders bevorzugt, wenn die erfindungsgemäße Zubereitung in Form einer O/W-Emulsion vorliegt.

Dabei ist es vorteilhaft im Sinne der vorliegenden Erfindung, wenn die Zubereitung einen oder mehrere der folgenden Emulgatoren bzw. Emulgatorkombinationen enthält: Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, PEG-5-Soyasterol, PEG-6 Sorbitan Beeswax, Glycerylstearat SE, Methylglucosesesquistearate, PEG-10 Hydrogenated Castor Oil, Sorbitanpalmitat, PEG-22/Dodecylglykol Copolymer, Polyglyceryl-2-PEG-4-Stearat, Sorbitanlaurat, PEG-4-Laurat, Polysorbat 61, Polysorbat 81, Polysorbat 65, Polysorbat 80, Triceteareth-4-Phosphat, Triceteareth-4 Phosphate und Sodium C₁₄₋₁₇ Alkyl Sec Sulfonat (Hostacerin CG von Hoechst), Glycerylstearat und PEG-100 Stearate (Arlacel 165 von ICI), Polysorbat 85, Trilaureth-4-Phosphat, PEG-35 Castor Oil, Sucrosestearat, Trioleth-8-Phosphat, C₁₂₋₁₅ Pareth-12, PEG-40 Hydrogenated Castor Oil, PEG-16 Soya Sterol, Polysorbat 80, Polysorbat 20, Polyglyceryl-3-methylglucose Distearat, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glycerides, Glyceryloleat und Propylenglykol, Glyceryllanolat, Polysorbat 60, Glycerylmyristat, Glycerylisostearat und Polyglyceryl-3 Oleat, Glyceryllaurat, PEG-40-Sorbitanperoleat, Laureth-4, Glycerinmonostearat, Isostearylglycerylether, Cetearyl Alcohol und Natriumcetearylsulfat, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-Isostearat, PEG-40-Sorbitanperoleat, PEG-40-Sorbitanperisostearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, Glycerylstearatcitrat, Cetylphosphat, Polyglycerolmethylglucosedistearat, Poloxamer 101, Kaliumcetylphosphat, Glycerylisostearat, Polyglyceryl-3-Diisostearate.

Besonders vorteilhaft im Sinne der Erfindung sind O/W-Emulgatoren aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die O/W-Emulgatoren werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren werden erfindungsgemäß vorzugsweise aus der folgenden Gruppe gewählt: Butyloctanol, Butyldecanol, Hexyloctanol, Hexyldecanol, Octyldodecanol, Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Die Gesamtpolarität der Lipidphase wird erfindungsgemäß wie folgt bestimmt:
- Meßgerät:: Ringtensiometer (z.B. Krüss K 10)
- Meßgröße:: spezifische Grenzflächenenergie = Grenzflächenspannung [Einheit: mN/m]
- Untere Grenze:: 5 mN/m

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl, Macadamia-, Avocado- oder Jojobaöl, Dialkylether wie z.B. Di-n-octylether sowie Dialkylcarbonate wie beispielsweise Di-n-octylcarbonat
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Hydrodispersionen oder Lipidispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäuretriglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyklischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, 2-Methyl-1,3-propandiol, Glycerin sowie insbesondere ein oder mehrere Verdickungsmittel.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feucht haltende Substanzen, Fette, Öle, Wachse oder andere übliche Bestandteile einer kosmetischen oder dermatologischen Formulierung wie Komplexbildner, Alkohole, Polyole, Polymere, Schaumstabilisatoren, Elektrolyte, organische Lösemittel oder Silikonderivate, Pflanzenextrakte, Vitamine, Parfümstoffe.

Insbesondere können erfindungsgemäß verwendete Wirkstoffkombinationen auch mit den in der Kosmetik bekannten Antioxidantien und/oder Radikalfängern kombiniert werden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Ester der 2-Cyano-3,3-Diphenylacrylsäure, bevorzugt Ethylhexyl-2-cyano-3,3-diphenylacrylat,
- Diethylhexyl-butamidotriazon, 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Weiterhin vorteilhafte UVA-Filter entstammen der Gruppe der Triazine, so z.B. das 2,4-Bis-{[4-(2-Ethyl-hexyloxy)-2-hydroxy]-phenyl}-6-(4-methoxyphenyl)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handels-bezeichnung Tinosorb® M). Ein vorteilhafter wasserlöslicher UVA-Filter stellt das 2'-bis-(1,4-Phenylen)-1H-benzimidazol-4,6-disulfonsäure-Natriumsalz dar (Handelsbezeichnung Neo Heliopan AP®).

Es können die für die UVB-Kombination verwendeten Mengen eingesetzt werden.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).
Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (N₂PO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Der erfindungsgemäße log P Wert wird dabei nach folgendem Programm berechnet:
- Hersteller:: Advanced Chemistry Development Inc. (ACD) 90 Adelaide Street West, Suite 702 Toronto / Canada
- Programm:: ACD / LogD Suite v. 4.5

Erfindungsgemäß ist die Verwendung der erfindungsgemäßen kosmetischen Zubereitung bzw. der erfindungsgemäßen Wirkstoffkombination aus Hyaluronsäure und Saponinen zur kosmetischen Prophylaxe und/oder Behandlung von Hautalterurgserscheinungen, insbesondere von Fältchen und Falten.

Erfindungsgemäß vorteilhaft ist die Verwendung einer erfindungsgemäßen Wirkstoffkombination (d.h. der Wirkstoffkombination aus 0,001 bis 3 Gewichts-% Hyaluronsäure und 0,01 bis 4 Gewichts-% Saponinen, bzw. 0,001 bis 3 Gewichts-% Hyaluronsäure und 0,01 bis 8 Gewichts-% Leguminosenextrakt, welcher 0,01 bis 99 Gewichts-% Saponine enthält) zur Herstellung eines Kosmetikums zur Behandlung von Hautalterungserscheinungen, insbesondere von Fältchen und Falten. Bei dem Pflanzenextrakt handelt es sich vorzugsweise um Sojaextrakt.

Erfindungsgemäß bevorzugt sind diese Verwendungen dann, wenn das Kosmetikum bzw. die kosmetische Zubereitung topisch auf der Haut angewendet wird.

Erfindungsgemäß bedeuten Prophylaxe und Behandlung im Rahmen dieser Offenbarung ausschließlich die kosmetische Prophylaxe und Behandlung und keinesfalls eine therapeutische Prophylaxe und Behandlung im Sinne des Patentrechtes.

Die erfindungsgemäßen Verwendungen finden insbesondere in Haut- oder Gesichtscremes, Haut- oder Gesichtslotionen sowie Tages- oder Nachtcremes oder -Iotionen Anwendung.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

### Beispiele 1-10: O/W-Cremes

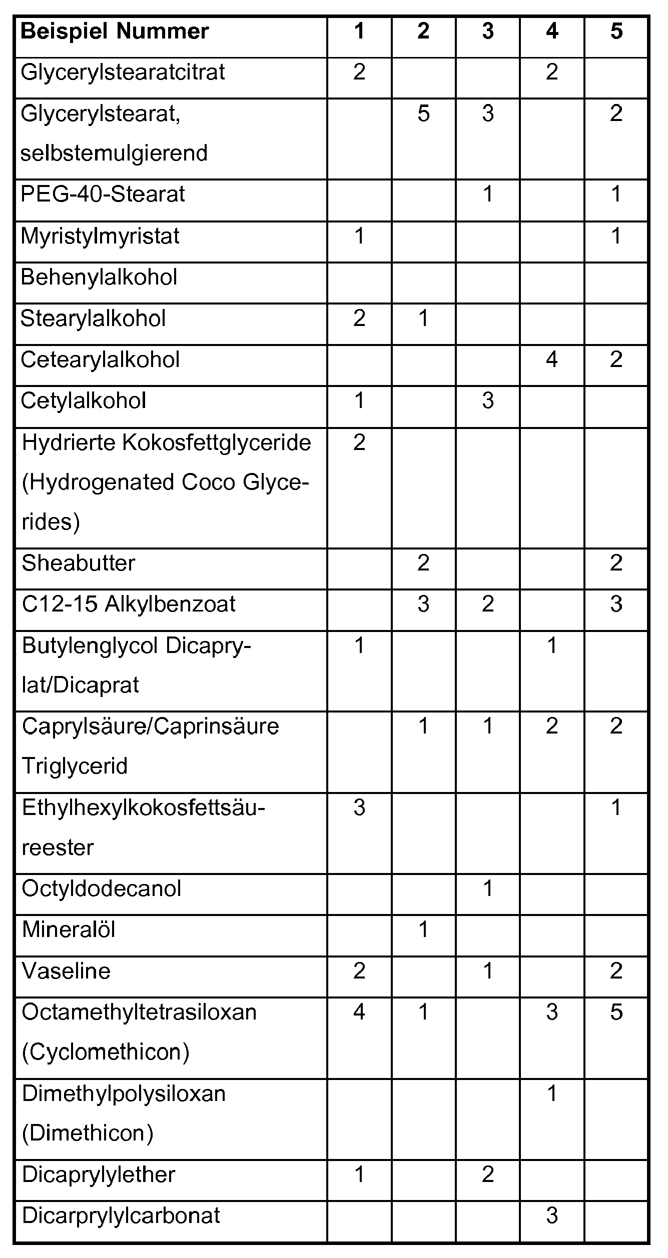

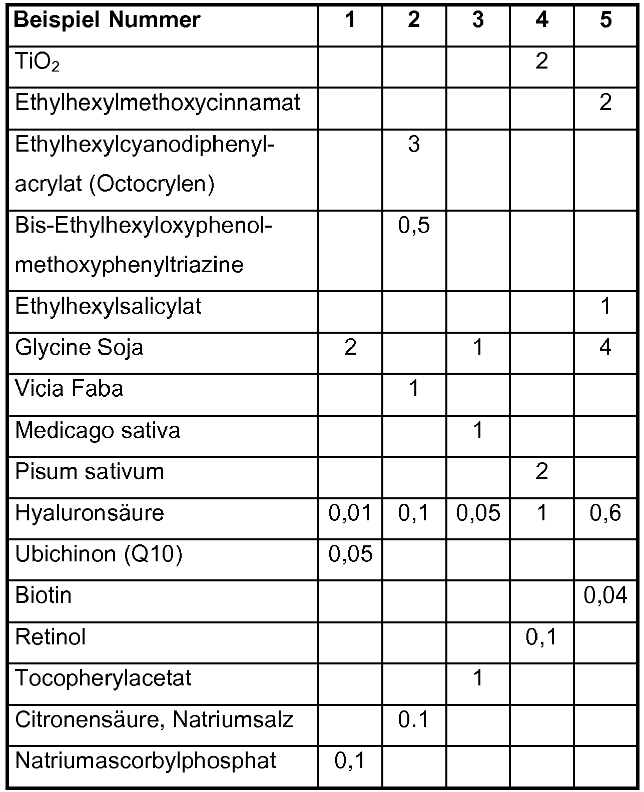

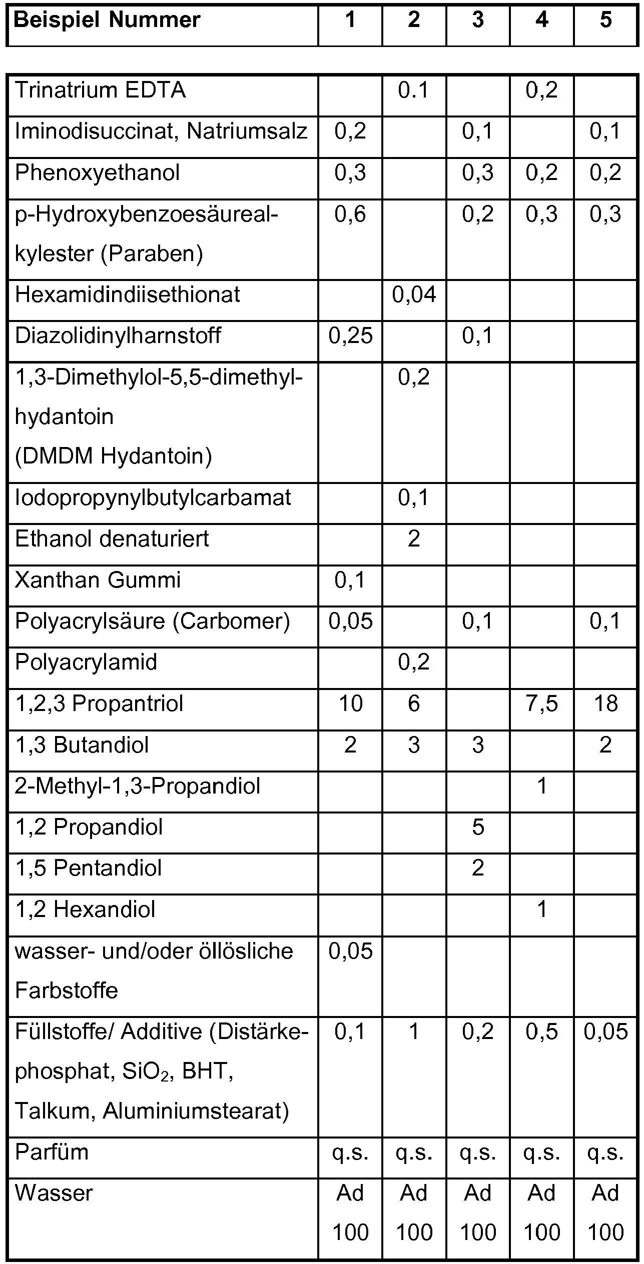

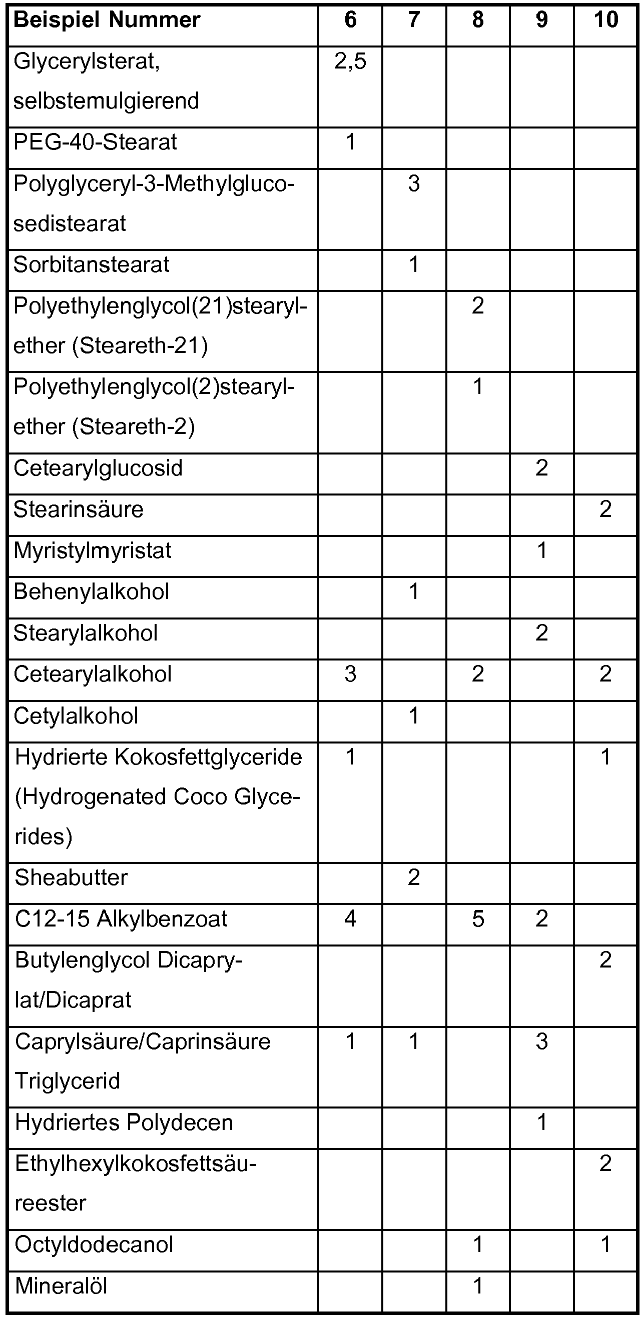

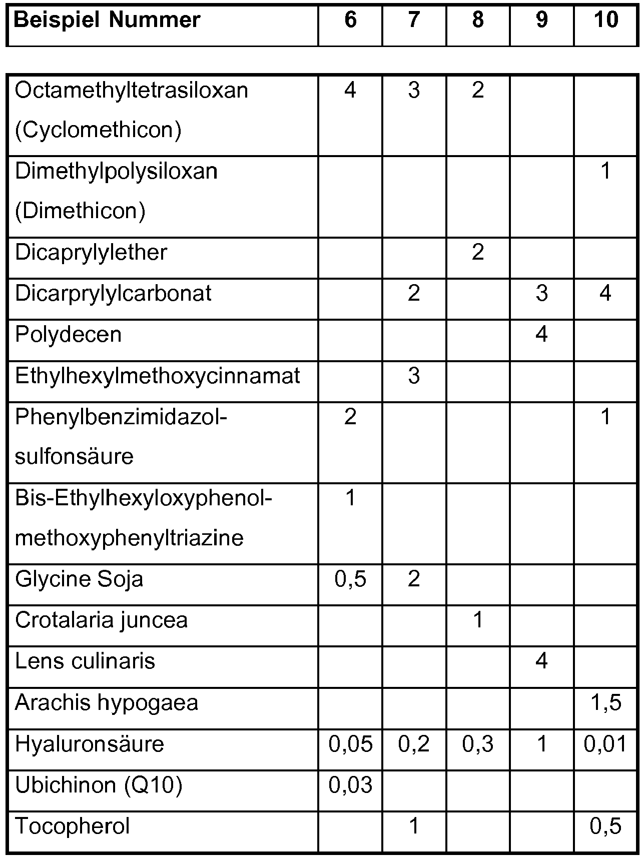

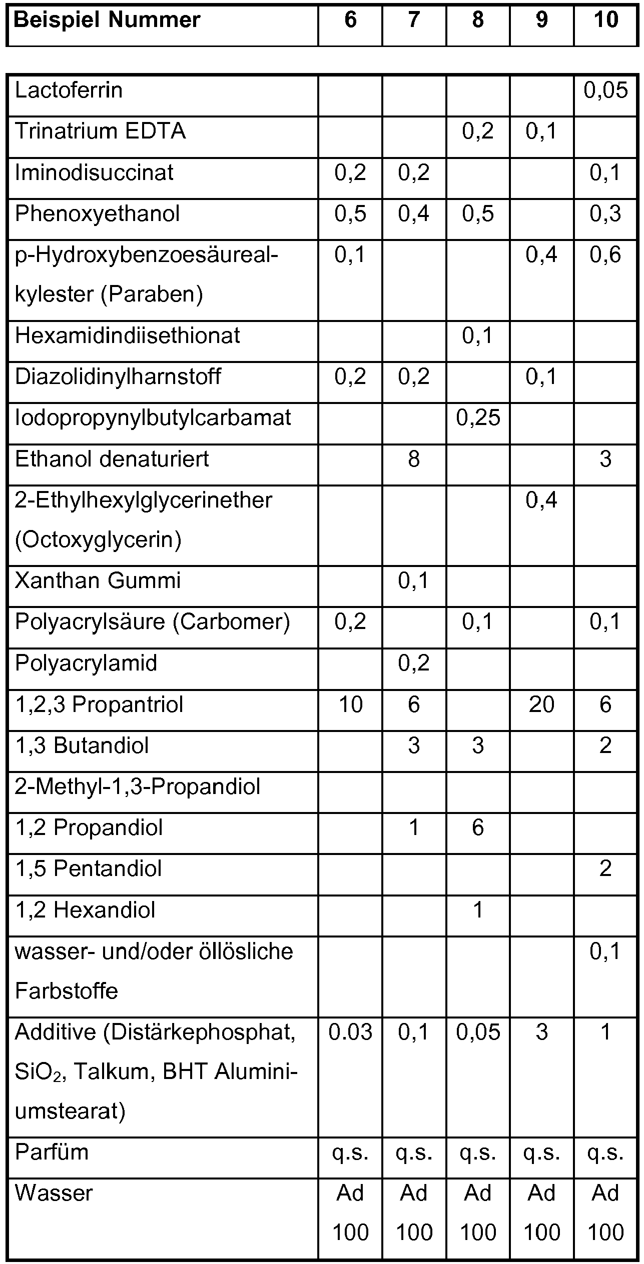

## Patentansprüche

1. Kosmetische Zubereitung in Form einer O/W-Emulsion enthaltend eine Wirkstoffkombination aus
0,001 bis 3 Gewichts-% Hyaluronsäure und
0,01 bis 4 Gewichts-% Saponinen,
wobei sich die Gewichtsangaben auf das Gesamtgewicht der Zubereitung beziehen, sowie enthaltend Wasser, ein Glycol mit einem log P Wert zwischen -3,6 und 1 und eine Lipidphase mit einer Gesamtpolarität von weniger als 30 mN/m.

2. Kosmetische Zubereitung nach Anspruch 1, **dadurch gekennzeichnet, dass** die Zubereitung Saponine aus Glycosiden von Triterpensapogeninen neben gegebenenfalls weiteren Saponinen aus Glycosiden von Steroidsapogeninen enthält.

3. Verwendung einer kosmetischen Zubereitung nach einem der vorhergehenden Ansprüche zur nicht therapeutischen Prophylaxe und/oder Behandlung von Hautalterungserscheinungen, insbesondere von Fältchen und Falten.

## Claims

1. Cosmetic preparation in the form of an O/W emulsion comprising an active ingredient combination of
0.001 to 3% by weight of hyaluronic acid and
0.01 to 4% by weight of saponins,
where the weight data refer to the total weight of the preparation, and also comprising water, a glycol with a log P value between -3.6 and 1 and a lipid phase with a total polarity of less than 30 mN/m.

2. Cosmetic preparation according to Claim 1, **characterized in that** the preparation comprises saponins from glycosides of triterpene sapogenins alongside optionally further saponins from glycosides of steroid sapogenins.

3. Use of a cosmetic preparation according to one of the preceding claims for the non-therapeutic prophylaxis and/or treatment of symptoms of skin ageing, in particular lines and wrinkles.

## Revendications

1. Préparation cosmétique sous la forme d'une émulsion H/E contenant une combinaison de substances actives constituée de
0,001 à 3 % en poids d'acide hyaluronique et
0,01 à 4 % en poids de saponines,
les indications en poids se rapportant au poids total de la préparation,
et contenant également de l'eau, un glycol avec une valeur log P comprise entre -3,6 et 1 et une phase liquide ayant une polarité totale inférieure à 30 mN/m.

2. Préparation cosmétique selon la revendication 1, **caractérisée en ce que** la préparation contient des saponines constituées de glycosides de sapogénines triterpène ainsi qu'éventuellement d'autres saponines constituées de glycosides de sapogénines stéroïdes.

3. Utilisation d'une préparation cosmétique selon l'une quelconque des revendications précédentes pour la prophylaxie non thérapeutique et/ou le traitement du vieillissement de la peau, en particulier de rides et de ridules.
